Europäisches Patentamt

European Patent Office

Office européen des brevets

(11) Publication number : **0 370 561 B1**

# EUROPEAN PATENT SPECIFICATION

(45) Date of publication of patent specification :
24.06.92 Bulletin 92/26

(51) Int. Cl.⁵ : **B01J 13/00**

(21) Application number : 89202883.8

(22) Date of filing : 14.11.89

(54) **Stained sols of non-metallic elements or compounds, their preparation and use.**

(30) Priority : 15.11.88 AU 25180/88

(43) Date of publication of application :
30.05.90 Bulletin 90/22

(45) Publication of the grant of the patent :
24.06.92 Bulletin 92/26

(84) Designated Contracting States :
AT BE CH DE ES FR GB GR IT LI LU NL SE

(56) References cited :
DE-C- 756 906
US-A- 2 329 147

(73) Proprietor : H.B.T. HOLLAND
BIOTECHNOLOGY B.V.
Niels Bohrweg 13
NL-2333 CA Leiden (NL)
Proprietor : AGROTECHNOLOGISCH
ONDERZOEKSINSTITUUT (ATO)
Haagsteeg 6
NL-6708 PM Wageningen (NL)

(72) Inventor : Wichers, Jan Herman
Tarthorst 703
NL-6708 JA Wageningen (NL)
Inventor : Van Es, Remco Maria
Henneland 25
NL-1541 NR Koog aan de Zaan (NL)
Inventor : Keizer, Gerrit Dirk
Vleugelnootlaan 34
NL-2382 EN Zoeterwoude Rijndijk (NL)
Inventor : Van Doorn, Albert Willem Jacob
Jacob Marislaan 60
NL-6813 JX Arnhem (NL)
Inventor : Van Gelder, Wilhelmus Martinus
Jozef
M.L. Kingplatsoen 2
NL-6671 BP Zetten (NL)

(74) Representative : Smulders, Theodorus A.H.J.,
Ir. et al
Vereenigde Octrooibureaux Nieuwe Parklaan
97
NL-2587 BN 's-Gravenhage (NL)

## Description

The invention relates to a process for the preparation of a sol of a non-metallic element or compound.

Such a process is known both from very old and from very recent publications. More than hundred years ago, Schulze, J. Pract. Chem. (2), 32, 390, 1885, described a preparation of selenium sols (i.e. dispersions of selenium particles in an aqueous liquid; the sols may also be referred to as hydrosols) by adding $SO_2$ to an aqueous solution of selenious acid ($H_2SeO_3$). In 1902, Gutbier, Z. Anorg. Chemie 32, 52, 1902, described a preparation of tellurium sols by treating an aqueous solution of telluric acid ($H_6TeO_6$) with hydrazine hydrate. Gutbier, Z. Anorg. Chemie 32, 106, 1902, also described a preparation of selenium sols by using hydrazine hydrate as reducing agent. Brintzinger, Koll. Zeitschr. 78, 22, 1937, disclosed a process for the preparation of selenium, tellurium, gold, palladium, platinum, silver, molybdenum and tungsten sols by reduction (for example of $SeO_2$) with ascorbic acid or isoascorbic acid as a reducing agent. US-A-2329147 and DE-A-756906 disclose stained sols of metals, especially of heavy and precious metals.

More recent publications are concerned with a particularly interesting use of sols, i.e. for the labelling of a component of the reaction between a specifically-binding substance and a corresponding bindable substance, in particular use of a sol as a label for a component of the reaction between a specifically-binding substance and a corresponding bindable substance in a method for assaying, detecting or measuring the presence and/or quantity of a component of said reaction in a test sample. In EP-A-0 007 654 (Akzo N.V., published Feb. 6, 1980) the use and preparation of sols of a metal or metal compound, in particular gold and silver sols, is described. EP-A-0 032 270 (Akzo N.V., published July 22, 1981) discloses the use and preparation of sols of a hydrophobic dye or pigment. The use and preparation of sols of non-metallic elements, such as selenium, tellurium and sulfur is taught in EP-A-0 298 368 (Abbott Laboratories, publ. Jan. 11, 1989) and in Dutch patent application NL-A-87.02769 and the corresponding part of EP-A-0 321 008 (in the name of H.B.T. Holland Biotechnology B.V., publication June 21, 1989).

The utility of a sol as a marker system for e.g. immuno-chemical reactions (for example in dipstick assays) generally requires a good visibility or, in more general terms, a good detectability of the sol particles. Some kinds of sols are not quite satisfactory in this respect because they are either not colored or only weakly colored. Examples of sols whose utility would be improved if they would have a more clearly detectable color are elemental sulfur sols, sols of the compound $SiO_2$, elemental selenium sols, and elemental copper sols.

The present invention provides a solution of this problem with uncolored or weakly colored sols and, in addition, creates new possibilities by a process for the preparation of a sol of a non-metallic element or compound, which process is characterized in that the sol is formed in a medium which contains at least one dye in solution to obtain a stained sol.

It should be understood that the words "a sol of a non-metallic element or compound" refer to a dispersion (preferably an aqueous dispersion, but alcoholic dispersions and dispersions in other organic liquids are not excluded) of particles substantially consisting of a non-metallic element or compound, without any intention to exclude that the sol particles are not fully homogeneous and, for example, contain a minor amount of the non-metallic element in the form of a chemical compound, i.e. the particles in a selenium sol may contain some selenium oxide, but most of the selenium will be present in the elemental form. Preferably at least 60%, more preferably at least 70% and most preferably substantially all of the selenium will be present in the elemental form. The same applies to sols of other elements, such as sulfur and, tellurium sols, while sol particles of a chemical compound, such as $SiO_2$ may contain minor amounts of other compounds or elements.

Of course, it is inherent to the words "a sol of a non-metallic element or compound" that the element or compound concerned is an element or compound which can exist in the form of a sol. Chemical elements such as hydrogen, helium, oxygen, etc., and chemical compounds such as sulfur dioxide and nitrogen oxides do not satisfy this inherent requirement and are consequently not covered by the words "element" and "compound" as used herein. As far as non-metallic elements are concerned, the invention is particularly concerned with sols such as selenium, sulfur, tellurium, arsenic and phosphorus sols. Most preferably, the invention relates to a process for preparing a selenium or sulfur sol. As far as chemical compounds are concerned, the invention is especially concerned with inorganic compounds. The most preferred embodiment of the invention, however, is a process for the preparation of stained silicium dioxide sols.

According to the invention, the process step in which the sol is formed is carried out in the presence of a dye which has been dissolved in the reaction medium. As the sols are usually prepared in aqueous media, the dye should preferably be soluble in a polar solvent such as water, alcohol, or acetone. It is preferred to use a dye carrying a nett negative charge when a sol is prepared which contains nett positively charged sol particles, and to use a dye carrying a nett positive charge when a sol is prepared which contains nett negatively charged sol particles. Other charge combinations, such as the addition of a negatively charged dye to a negatively charged sol, are not excluded, however. The use of a dye which carries the opposite charge compared to the sol particles to be formed is beneficial to obtain an irreversible and strong binding of the dye to the sol particles. Indeed, it was experimentally found impossible to remove the dye from the sol particles, either by washing or by dialysing. In the case of flocculation of the stained sols, the dye remains tightly bound to the sol particles and is flocculated therewith; no dye leaves the flocculated particles.

Particular embodiments of the invention are characterized in that a dye, selected from the group consisting of fluorescent rhodamine isothiocyanate, Brilliant Cresyl Blue, Malachite green, and Oracet Blue (Chroma, Stuttgart, West Germany), is used.

The advantages of stained sols obtainable by the process of the invention are numerous. First of all, the invention makes it possible to provide originally uncolored sols with very bright colors, thus creating or improvimg their utility as a marker system. For example, the invention makes it possible to provide $SiO_2$ sol particles, which are white by their own, with any desired color, such as red, blue, green, etc. In addition, the invention allows for the preparation and use of one kind of sol particle in different colors. When two or more differently stained $SiO_2$ sols having clearly distinctive colors are prepared and each is coupled to a different binding substance, e.g. different monoclonal antibodies, two or more agents can be recognized/detected on the same dipstick. Also, one of such differently stained sols could serve as a control of the correct reaction. Another advantage of the invention is the fact that fluorescent dyes can be used, since it is a known fact that the use of fluorescent dyes can lead to a higher assay sensitivity compared to the normal dyes.

In view of these unexpected and highly advantageous properties of sols prepared by the process of the invention, the invention also extends to sols of a non-metallic element or compound, which are obtainable by the process of the invention.

As is evident from the cited publications EP-A-0 007 654, EP-A-0 032 270, EP-A-0 298 368 and NL-A-87.02769, sols of non-metallic elements or compounds may be used for the labelling of a component of the reaction between a specifically-binding substance and a corresponding bindable substance. The invention extends to such a use, in particular to the use of a sol of a non-metallic element or compound, which is obtainable by the process according to the invention, as a label for a component of the reaction between a specifically-binding substance (e.g. protein, DNA, RNA, polysaccharide) and a corresponding bindable substance in a method for assaying, detecting or measuring the presence and/or quantity of a component of said reaction in a test sample.

The invention will be explained in greater detail by means of the following examples of preferred embodiments thereof. It should be noted, however, that the examples are not intended to limit the scope of the invention but merely serve to illustrate the invention. The operations described hereunder were performed at room temperature, unless otherwise stated.

Example 1

(a) Preparation of a $SiO_2$ sol stained with fluorescent rhodamine isothiocyanate

100 mg Rhodamine isothiocyanate (RITC, Sigma) was dissolved in 862 ml absolute ethanol (Merck). To this solution, 74 ml of a 25% aqueous $NH_3$ solution (Merck) was added, using a magnetic stirrer for mixing both solutions. Then 64 ml tetraethyl orthosilicate (Merck) was added quickly with vigorous stirring. The initial clear purplish/red solution becomes visibly opalescent after about 7 minutes.

After 120 minutes, the sol was purified by dialysis (by means of an artificial kidney). During the dialysis, the pH of the sol was decreased from pH 10.0 to pH 6.5. The dialysis was terminated as soon as the dialysate no longer contained spectrophotometrically measurable quantities of RITC: ($A_{560\,nm}^{1\,cm} = 0$).

Thus, an opalescent, purple $SiO_2$ sol contained in an aqueous medium was formed. The average diameter of the spherical $SiO_2$ particles was $156 \pm 8$ nm.

(b) Adsorption of a monoclonal mouse IgG to colloidal RITC/$SiO_2$

Prior to use, the sol was diluted to $A(\lambda = 560$ nm$) = 1.0$. The pH of 5 ml of the RITC/$SiO_2$ sol was adjusted to pH 7.0 by means of 0.1 M $K_2CO_3$. Mouse ascites (50 µl) was added under gentle stirring to said 5 ml neutralized RITC/$SiO_2$ sol. After an incubation of 15 minutes, the volume was made up with water to 15 ml.

The formed RITC/$SiO_2$-IgG(ascites) conjugate was centrifuged at 1300xg (4°C) for 5 minutes. The supernatant was collected on a suction filter and the loose pellet containing the conjugate was re-suspended in 500 µl 5 mM NaCl solution.

(c) <u>Test procedure and results</u>

A concentration series of goat anti-mouse polyclonal IgG (1280 ng to 2.5 pg) was spotted onto nitrocellulose test strips[1]. As a negative control, a concentration series of β-galactosidase (1500 ng to 2.0 pg) was included.

Cross-incubation[2] of the various strips was effected both with RITC/SiO$_2$-IgG(ascites) conjugate and with unprotected RITC/SiO$_2$ sol. The following results were obtained.

Goat anti-mouse spots were specifically stained by the RITC/SiO$_2$-IgG(ascites) conjugate up to 40 ng. Goat anti-mouse spots incubated with unprotected RITC/SiO$_2$ sol did not show any specific staining.

β-galactosidase spots did not exhibit any specific staining either with the RITC/SiO$_2$-IgG(ascites) conjugate or with unprotected RITC/SiO$_2$ sol.

Note (1): the test strips were made of nitrocellulose paper having a pore diameter of 0.45 μm and being cut to a size of 1.2 cm x 5.5 cm. The spots applied to the test strips had a volume of 1 μl. After spotting the concentration series, the strips were dried at 37°C for 20 minutes. Immediately thereafter, the bare parts of the strips (i.e. the parts that were not covered) were blocked with a 3% Bovine Serum Albumine (BSA) solution in Phosphate Buffered Saline (PBS) at 37°C. After 20 minutes, the strips were dried at 37°C and were then ready for use.

Note (2): the test strips were incubated in a 1:50 dilution of the probe in PBS + 1% BSA for at most 2 hours. During the incubation, the strips and incubation mixture were agitated on a shaker in polypropylene tubes. The sensitivity of a probe was determined by the lowest visually detectable concentrations of spotted (immobilized) protein in non-optimized systems.

## Example 2

<u>Preparation of a SiO$_2$ sol stained blue with Brilliant Cresyl Blue</u>

100 mg Brilliant Cresyl Blue (BCB, B.D.H.) was dissolved in 862 ml absolute ethanol (Merck). To this solution, 74 ml of a 25% aqueous NH$_3$ solution (Merck) was added, using a magnetic stirrer for mixing both solutions. Then 64 ml tetraethyl orthosilicate (Merck) was added quickly with vigorous stirring. The initial clear purple solution becomes visibly opalescent after about 7 minutes.

After 120 minutes, the sol was purified by dialysis (by means of an artificial kidney). During the dialysis, the pH of the sol was decreased from pH 10.0 to pH 6.5 which resulted in a color change from purple to blue. The dialysis was terminated as soon as the dialysate no longer contained spectrophotometrically measurable quantities of BCB: ($A_{560\,nm}^{1\,cm} = 0$).

Thus, an opalescent, blue SiO$_2$ sol contained in an aqueous medium was formed. The average diameter of the spherical SiO$_2$ particles was about 150 nm.

## Example 3

<u>Preparation of a SiO$_2$ sol stained green with Malachite Green</u>

100 mg Malachite Green (MG, Sigma) was dissolved in 862 ml absolute ethanol (Merck). To this solution, 74 ml of a 25% aqueous NH$_3$ solution (Merck) was added, using a magnetic stirrer for mixing both solutions. Then 64 ml tetraethyl orthosilicate (Merck) was added quickly with vigorous stirring. The initial clear colorless solution became visibly opalescent after about 7 minutes.

After 120 minutes, the sol was purified by dialysis (by means of an artificial kidney). During the dialysis, the pH of the sol was decreased from pH 10.0 to pH 6.5; as a result, the opalescent colorless sol turned dark blue-green. The dialysis was terminated as soon as the dialysate no longer contained spectrophotometrically measurable quantities of MG: ($A_{560\,nm}^{1\,cm} = 0$).

Thus, an opalescent, green SiO$_2$ sol contained in an aqueous medium was formed. The average diameter of the spherical SiO$_2$ particles was about 150 nm.

## Example 4

<u>(a) Preparation of a selenium sol stained with Oracet Blue</u>

Oracet Blue (0.005 g) was dissolved in 1 ml ethanol. After 10 minutes, 9 ml demineralised water was added, followed by a filtration of the solution (solution A).

A second stock solution was prepared by dissolving 0.005 g Oracet Blue in 1 ml acetone. After 10 min, 9 ml demi-water was added, followed by a filtration of the solution (solution B).

$SeO_2$ was dissolved in demi-water to a concentration of 0.2 g/l, and $NaBH_4$ was dissolved in demineralised water to a concentration of 25 g/l.

Several sols were prepared under vigorous stirring by adding 1 ml of the above $NaBH_4$ solution and different amounts of Oracet Blue and demineralised water to 100 ml of the above $SeO_2$ solution. Data on the sols prepared are given in Table I.

TABLE I

| dye solution (ml) | demi-water (ml) | sol color |
|---|---|---|
| 0 | 3 | orange-red |
| 1 (A) | 2 | brown-red |
| 2 (A) | 1 | brown |
| 3 (A) | 0 | dark brown |
| 1 (B) | 2 | blue-green |
| 3 (B) | 0 | dark brown |

After an incubation of 30 minutes, the sols were purified by means of dialysis. The sols did not change color upon dialysis. Moreover, addition of $NaBH_4$ to Oracet Blue alone did not result in a change of color, indicating that the color change from orange-red to brown of the selenium sol was due to the formation of a stained selenium sol.

(b) Adsorption of IgG to colloidal stained Se-sols

The pH of 50 ml sol was adjusted to pH 7.5 by addition of 0.01 M $K_2CO_3$. Then, 100 µl polyclonal rabbit antibodies (serum) directed against nortestosteron were added. Coupling took place at room temperature for 10 minutes. The mixture was then centrifuged at 4,600xg (4°C) for 10 minutes. The supernatant was removed and the pellet was suspended in 5 ml NaCl solution (1/20th part of the original volume). The anti-nortestosteron IgG-stained selenium probe thus obtained was used for detection purposes.

(c) Test procedure and results

Different amounts of conjugated BSA-nortestosteron were spotted onto nitrocellulose strips. After drying and blocking the nitrocellulose with a 2% BSA solution, the nitrocellulose strip was incubated with the stained sol. After a few minutes, dark brown spots became visible. Stained sols which had been coated with irrelevant rabbit sera did not stain the spots. The same procedure with unstained Se-sols resulted in less visible orange-red spots.

**Claims**

1. A process for the preparation of a sol of a non-metallic element or compound, characterized in that the sol is formed in a medium which contains at least one dye in solution to obtain a stained sol.

2. A process according to claim 1, characterized in that a dye is used, which is soluble in a polar solvent such as water, alcohol, or acetone.

3. A process according to claim 1 or 2, characterized in that a dye carrying a nett negative charge is used when a sol is prepared which contains nett positively charged sol particles, and that a dye carrying a nett positive charge is used when a sol is prepared which contains nett negatively charged sol particles.

4. A process according to claim 1, characterized in that a dye, selected from the group consisting of fluorescent rhodamine isothiocyanate, Brilliant Cresyl Blue, Malachite green, and Oracet Blue, is used.

5. A process according to any of claims 1 to 4, characterized in that a sol of the chemical compound $SiO_2$ is prepared.

6. A process according to any of claims 1 to 4, characterized in that a sol of the element selenium (Se) is prepared.

7. A process according to any of claims 1 to 4, characterized in that a sol of the element sulfur (S) is prepared.

8. A process according to any of claims 1 to 4, characterized in that a sol of the element tellurium (Te) is prepared.

9. A stained sol of a non-metallic element or compound, which is obtainable by the process of any of claims 1 to 8.

10. The use of a stained sol of a non-metallic element or compound, which is obtainable by the process of any of claims 1 to 8, for labeling a component of the reaction between a specifically-binding substance and a corresponding bindable substance.

11. The use of a stained sol of a non-metallic element or compound, which is obtainable by the process of any of claims 1 to 8, as a label for a component of the reaction between a specifically-binding substance and a corresponding bindable substance in a method for assaying, detecting or measuring the presence and/or quantity of a component of said reaction in a test sample.

**Patentansprüche**

1. Verfahren zur Herstellung eines Sols eines nicht-metallischen Elements oder einer nicht-metallischen Verbindung, dadurch gekennzeichnet, daß das Sol in einem Medium gebildet wird, das wenigstens einen Farbstoff in Lösung enthält, um ein gefärbtes Sol zu erhalten.

2. Verfahren nach Anspruch 1, dadurch gekennzeichnet, daß ein Farbstoff verwendet wird, der in einem polaren Lösungsmittel wie Wasser, Alkohol oder Aceton löslich ist.

3. Verfahren nach Anspruch 1 oder 2, dadurch gekennzeichnet, daß ein eine netto negative Ladung tragender Farbstoff verwendet wird, wenn ein Sol hergestellt wird, das netto positiv geladene Solpartikeln enthält, und daß ein eine netto positive Ladung tragender Farbstoff verwendet wird, wenn ein Sol hergestellt wird, das netto negativ geladene Solpartikeln enthält.

4. Verfahren nach Anspruch 1, dadurch gekennzeichnet, daß ein Farbstoff verwendet wird, der aus der Gruppe, bestehend aus fluoreszierendem Rhodamin-Isothiocyanat, Brilliant Cresyl Blue, Malachitgrün und Oracet Blue, gewählt ist.

5. Verfahren nach einem der Ansprüche 1 bis 4, dadurch gekennzeichnet, daß ein Sol der chemischen Verbindung $SiO_2$ hergestellt wird.

6. Verfahren nach einem der Ansprüche 1 bis 4, dadurch gekennzeichnet, daß ein Sol des Elements Selen (Se) hergestellt wird.

7. Verfahren nach einem der Ansprüche 1 bis 4, dadurch gekennzeichnet, daß ein Sol des Elements Schwefel (S) hergestellt wird.

8. Verfahren nach einem der Ansprüche 1 bis 4, dadurch gekennzeichnet, daß ein Sol des Elements Tellur (Te) hergestellt wird.

9. Gefärbtes Sol eines nicht-metallischen Elements oder einer nicht-metallischen Verbindung, das durch das Verfahren nach einem der Ansprüche 1 bis 8 erhältlich ist.

10. Verwendung eines gefärbten Sols eines nicht-metallischen Elements oder einer nicht-metallischen Verbindung, das durch das Verfahren nach einem der Ansprüche 1 bis 8 erhältlich ist, zum Markieren einer Komponente der Reaktion zwischen einer spezifisch bindenden Substanz und einer entsprechend bindbaren Substanz.

11. Verwendung eines gefärbten Sols eines nicht-metallischen Elements oder einer nicht-metallischen Verbindung, das durch das Verfahren nach einem der Ansprüche 1 bis 8 erhältlich ist, als Markierung für eine Komponente der Reaktion zwischen einer spezifisch bindenden Substanz und einer entsprechend bindbaren Substanz in einem Verfahren zum Prüfen, Ermitteln oder Messen der Anwesenheit und/oder Quantität einer Komponente der genannten Reaktion in einer Probe.

**Revendications**

1. Procédé de préparation d'un sol d'un élément non-métallique ou d'un composé d'un élément non-métallique, **caractérisé en ce que** le sol est formé dans un milieu qui contient en solution au moins une teinture, pour obtenir un sol coloré.

2. Procédé selon la revendication 1, **caractérisé en ce que** l'on recourt à une teinture soluble dans un solvant polaire tel que l'eau, l'alcool ou l'acétone.

3. Procédé selon la revendication 1 ou 2, **caractérisé en ce que** l'on recourt à une teinture possédant une charge négative nette lorsque l'on prépare un sol contenant des particules de sol dont la charge nette est positive, et à une teinture possédant une charge positive nette lorsqu'on prépare un sol contenant des particules de sol dont la charge nette est négative.

4. Procédé selon la revendication 1, **caractérisé en ce que** l'on recourt à une teinture choisie parmi le groupe reprenant l'isothiocyanate de rhodamine fluorescent, le bleu de crésyl brillant, le vert de malachite et le bleu d'oracet.

5. Procédé selon l'une quelconque des revendications 1 à 4, **caractérisé en ce que** l'on prépare un sol du composé chimique $SiO_2$.

6. Procédé selon l'une quelconque des revendications 1 à 4, **caractérisé en ce que** l'on préparer un sol de l'élément sélénium (Se).

7. Procédé selon l'une quelconque des revendications 1 à 4, **caractérisé en ce que** l'on préparer un sol de l'élément soufre (S).

8. Procédé selon l'une quelconque des revendications 1 à 4, **caractérisé en ce que** l'on préparer un sol de l'élément tellure (Te).

9. Sol coloré d'un élément non-métallique ou d'un composé d'un élément non-métallique, pouvant être obtenu par le procédé selon l'une quelconque des revendications 1 à 8.

10. Recours à un sol coloré d'un élément non-métallique ou d'un composé d'un élément non-métallique, qui peut être obtenu par recours au procédé selon l'une quelconque des revendications 1 à 8, en vue de marquer un des composants d'une réaction entre une substance ligante non spécifique et une substance correspondante pouvant être liée.

11. Recours à un sol coloré d'un élément non-métallique ou d'un composé d'un élément non-métallique, qui peut être obtenu par recours au procédé selon l'une quelconque des revendications 1 à 8, comme un marquage d'un des composants d'une réaction entre une substance ligante spécifique et une substance correspondante pouvant être liée, selon une méthode visant à contrôler, détecter ou mesurer la présence et/ou la quantité d'un composé de ladite réaction dans un échantillon à tester.